(19) Europäisches Patentamt
European Patent Office
Office européen des brevets

(11) **EP 4 230 190 A1**

(12) **EUROPEAN PATENT APPLICATION**
published in accordance with Art. 153(4) EPC

(43) Date of publication:
23.08.2023 Bulletin 2023/34

(21) Application number: 21880051.4

(22) Date of filing: 11.10.2021

(51) International Patent Classification (IPC):
*A61K 8/37* (2006.01)        *A61K 8/06* (2006.01)
*A61K 8/19* (2006.01)        *A61K 8/63* (2006.01)

(52) Cooperative Patent Classification (CPC):
A61K 8/06; A61K 8/19; A61K 8/37; A61K 8/63;
A61K 8/86; A61K 8/891; A61K 8/92; A61Q 1/00

(86) International application number:
PCT/JP2021/037567

(87) International publication number:
WO 2022/080312 (21.04.2022 Gazette 2022/16)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 15.10.2020 JP 2020174262

(71) Applicant: Kao Corporation
Chuo-ku
Tokyo 103-8210 (JP)

(72) Inventor: TATEBE, Momoko
Odawara-shi, Kanagawa 250-0002 (JP)

(74) Representative: Hoffmann Eitle
Patent- und Rechtsanwälte PartmbB
Arabellastraße 30
81925 München (DE)

(54) **WATER-IN-OIL EMULSION COMPOSITION**

(57)    A water-in-oil emulsion composition comprising the following components (A), (B), (C), (D), and (E): (A) a lipophilic nonionic surfactant; (B) an oil agent which is solid or semisolid at 25°C; (C) a nonvolatile oil which is liquid at 25°C; (D) a carbonate; and (E) water.

**Description**

Field of the Invention

The present invention relates to a water-in-oil emulsion composition.

Background of the Invention

For makeup cosmetics such as foundations, it is necessary to firmly adhere the cosmetic to the skin in order to cover the pores.

For example, Patent Literature 1 states that a water-in-oil emulsion cosmetic containing a composite powder containing particles in which the surfaces of silicone rubber particles are coated with a silicone resin, and a nonvolatile oil agent other than a silicone oil makes the skin bright, is excellent in extensibility and close adhesiveness, and exhibits a pore hiding effect.

Meanwhile, sunscreen cosmetics containing carbonates are known.

For example, Patent Literature 2 describes a sunscreen cosmetic containing an inorganic ultraviolet scattering agent containing a hydrophobic fine particle metal oxide, tubular basic magnesium carbonate, and an oil, and Patent Literature 3 describes a cosmetic composition having UV-A and/or UV-B protection containing at least one inorganic UV filter and surface-reacted calcium carbonate.

In these cosmetics, carbonates are used to improve the ultraviolet protection effect.

(Patent Literature 1) JP-A-2014-172872
(Patent Literature 2) JP-A-2011-236182
(Patent Literature 3) JP-A-2020-506953

Summary of the Invention

The present invention relates to a water-in-oil emulsion composition comprising the following components (A), (B), (C), (D), and (E):

(A) a lipophilic nonionic surfactant;
(B) an oil agent which is solid or semisolid at 25°C;
(C) a nonvolatile oil which is liquid at 25°C;
(D) a carbonate; and
(E) water.

Detailed Description of the Invention

Conventional emulsion cosmetics tend to give a sliding feeling on the skin during application, an actual feeling that the cosmetic is sufficiently adhered to the skin is insufficient, and the adhesion of the cosmetic to the pores is also weak, and the pores cannot be sufficiently covered. Another problem is that a sticky feeling remains on the skin after application.

The present inventors found that a water-in-oil emulsion composition which solves the above problems is obtained by using a carbonate in combination with a lipophilic nonionic surfactant, an oil agent which is solid or semisolid at 25°C, and a nonvolatile oil which is liquid at 25°C.

With the water-in-oil emulsion composition of the present invention, the sliding feeling is reduced during application, the feeling of close adhesion to the skin after application is improved, and the pores can be sufficiently covered, and moreover the sticky feeling on the skin after application is reduced.

The sliding feeling indicates that when a water-in-oil emulsion cosmetic is taken on a fingertip or the like and applied to the face, there is a feeling that the water-in-oil emulsion cosmetic spreads for a long period of time on the skin as if a liquid oil agent were spread, and there is no actual feeling that the water-in-oil emulsion cosmetic spreads while adhering to the skin. The feeling of close adhesion indicates that a water-in-oil emulsion composition attached to the skin after application follows the movement of the skin but does not come off the surface of the skin even if the skin of the face is moved by a change in expression, or the like; and the water-in-oil emulsion composition does not gather partially in the form of large wrinkles, small wrinkles, and the like; and the water-in-oil emulsion composition adheres firmly.

The component (A) lipophilic nonionic surfactant used in the present invention preferably has an HLB of from 1 to 8, more preferably from 2 to 7, and further more preferably from 3 to 6 from the viewpoint of reducing the sliding feeling of the water-in-oil emulsion composition on the skin during application, improving the feeling of close adhesion to the skin after application, and obtaining a stable water-in-oil emulsion composition.

Here, HLB (Hydrophilic-Lipophilic Balance) represents the molecular weight of the hydrophilic group moiety in the total molecular weight of the surfactant and is obtained by Griffin's equation. When the component (A) is composed of two or more nonionic surfactants, the HLB of the surfactant mixture is obtained as follows. The HLB of the surfactant mixture is the arithmetic mean of the HLB values of the nonionic surfactants based on their blend ratio.

$$\texttt{HLB for mixture} = \Sigma(\texttt{HLBx} \times \texttt{Wx})/\Sigma\texttt{Wx}$$

HLBx represents the HLB value of a nonionic surfactant X.

Wx represents the mass (g) of the nonionic surfactant X having the value of HLBx.

Examples of the component (A) lipophilic nonionic surfactant include glycerin fatty acid esters, propylene glycol fatty acid esters, sorbitan fatty acid esters such as sorbitan monoisostearate, sorbitan monooleate, sorbitan sesquistearate, and sorbitan sesquioleate, polyoxyethylene castor oil, polyoxyethylene hydrogenated castor oil, polyoxyethylene hydrogenated castor oil fatty acid esters, polyethylene glycol fatty acid esters, alkyl glyceryl ethers, alkyl polyglyceryl ethers, polyoxyethylene alkyl ethers, polyoxyethylene alkyl ether fatty acid esters, polyoxyethylene alkylamines, and silicone-based surfactants such as polyether-modified silicones, alkyl-modified polyether-modified silicones, and alkyl chain-co-modified polyether-modified silicones, such as polyoxyethylene-methylpolysiloxane copolymers and poly(oxyethylene-oxypropylene) methylpolysiloxane copolymers.

Among these, at least one or more selected from the group consisting of sorbitan fatty acid esters and silicone-based surfactants are preferably included, at least one or more selected from the group consisting of sorbitan fatty acid esters and polyether-modified silicones are more preferably included, at least one or more selected from the group consisting of sorbitan monoisostearate, sorbitan monooleate, sorbitan sesquistearate, and polyether-modified silicones are further more preferably included, and at least one or more selected from the group consisting of sorbitan monoisostearate and polyoxyethylene-methylpolysiloxane copolymers are even more preferably included, from the viewpoint of reducing the sliding feeling of a water-in-oil emulsion composition on the skin during application, improving the feeling of close adhesion to the skin after application, and obtaining a stable water-in-oil emulsion composition.

A sorbitan fatty acid ester and a silicone-based surfactant are preferably used in combination.

As the component (A) lipophilic nonionic surfactant, a commercial product can be used. Examples of sorbitan monoisostearate include Span 120 (HLB 4.7) (manufactured by Croda). Examples of the polyoxyethylene-methylpolysiloxane copolymers include KF-6015 (HLB 4.5), KF-6017 (HLB 4.5), and KF-6028 (HLB 4) (all manufactured by Shin-Etsu Chemical Co., Ltd.), and SH3775M (HLB 5) (manufactured by Dow Corning Toray Co., Ltd.). Examples of the poly(oxyethylene-oxypropylene) methylpolysiloxane copolymers include KF-6012 (HLB 7) (manufactured by Shin-Etsu Chemical Co., Ltd.), and DOWSIL BY 22-008 M (HLB 2) and ES-5226 DM formulation Aid (HLB 2) (all manufactured by Dow Corning Toray Co., Ltd.). Examples of the alkyl-modified polyether-modified silicones include KF-6038 (HLB 3) (manufactured by Shin-Etsu Chemical Co., Ltd.) and ABIL EM 90 (HLB 5) (manufactured by Evonik).

One or more lipophilic nonionic surfactants, components (A), can be used, and the content is preferably 1% by mass or more, more preferably 1.5% by mass or more, and further more preferably 2% by mass or more, and preferably 8% by mass or less, more preferably 6.5% by mass or less, and further more preferably 5.5% by mass or less in the total composition from the viewpoint of reducing the sliding feeling of the water-in-oil emulsion composition on the skin during application, improving the feeling of close adhesion to the skin after application, and obtaining a stable water-in-oil emulsion composition. The content of the component (A) is preferably from 1 to 8% by mass, more preferably from 1.5 to 6.5% by mass, and further more preferably from 2 to 5.5% by mass in the total composition.

The component (B) is an oil agent which is solid or semisolid at 25°C.

Being solid or semisolid at 25°C means that the viscosity at 25°C is greater than 10,000 mPa·s.

Here, the viscosity is measured with a B type viscometer (model TVB-10, manufactured by TOKI SANGYO CO., LTD.), rotor No. 4, 12 rpm, and 60 s (rotor No. 4, 3 rpm, and 60 s for 50,000 mmPa.s or more).

The oil agent which is solid at 25°C is not limited as long as it is usually used in cosmetics. An ester wax, a hydrocarbon wax, or the like can be used. Examples of the oil agent which is solid at 25°C containing an ester wax include animal waxes and vegetable waxes, and examples of the oil agent which is solid at 25°C containing a hydrocarbon wax include mineral waxes and synthetic waxes. More specifically, examples of the oil agent which is solid at 25°C containing an ester wax include shea butter, rice bran wax, carnauba wax, candelilla wax, and beeswax, which are vegetable waxes, and examples of the oil agent which is solid at 25°C containing a hydrocarbon wax include ceresin, paraffin waxes, microcrystalline waxes, polyethylene waxes, and polyolefin waxes.

Examples of the oil agent which is semisolid at 25°C include:

cholesterol derivatives such as cholesteryl isostearate, cholesteryl hydroxystearate, cholesteryl macadamiate, and di(cholesteryl-behenyl-octyldodecyl) N-lauroyl-L-glutamate;

phytosterol derivatives such as di(phytosterylbehenyl-2-octyldodecyl) N-lauroyl-L-glutamate, di(phytosteryl-2-octyldodecyl) N-lauroyl-L-glutamate, phytosteryl isostearate, phytosteryl oleate, and (phytosteryl/isostearyl/cetyl/stearyl/behenyl) dimer dilinoleate;

dipentaerythritol fatty acid esters such as dipentaerythritol hexaoxystearate and dipentaerythritol rosinate;

triglycerides such as tri(caprylic-capric-myristic-stearic acid) glyceride, glyceryl trilanolate, and glyceryl tribehenate;

partially hydrogenated triglycerides such as hydrogenated oils, lanolin, soft lanolin fatty acid, lanosterol derivatives, Vaseline, and dimer dilinoleic acid hydrogenated castor oil.

The component (B) preferably contains at least one or more selected from the group consisting of vegetable waxes, cholesterol derivatives, phytosterol derivatives, triglycerides, lanolin, Vaseline, and dimer dilinoleic acid hydrogenated castor oil, more preferably at least one or more selected from the group consisting of vegetable waxes, cholesterol derivatives, phytosterol derivatives, triglycerides, lanolin, and Vaseline, further more preferably at least one or more selected from the group consisting of vegetable waxes, phytosterol derivatives, and triglycerides, even more preferably at least one or more selected from the group consisting of vegetable waxes and triglycerides, and especially preferably a triglyceride from the viewpoint of reducing the sliding feeling of the water-in-oil emulsion composition on the skin during application, improving the feeling of close adhesion to the skin after application and the pore covering ability, and reducing the sticky feeling.

The component (B) preferably contains at least one or more selected from the group consisting of shea butter, (phytosteryl/isostearyl/cetyl/stearyl/behenyl) dimer dilinoleate, and glyceryl tribehenate.

One or more components (B) can be used, and the content is preferably 0.01% by mass or more, more preferably 0.03% by mass or more, and further more preferably 0.07% by mass or more, and preferably 8% by mass or less, more preferably 5% by mass or less, and further more preferably 3% by mass or less in the total composition from the viewpoint of reducing the sliding feeling of the water-in-oil emulsion composition on the skin during application, improving the feeling of close adhesion to the skin after application and the pore covering ability, and reducing the sticky feeling. The content of the component (B) is preferably from 0.01 to 8% by mass, more preferably from 0.03 to 5% by mass, and further more preferably from 0.07 to 3% by mass in the total composition.

The component (C) is a nonvolatile oil which is liquid at 25°C.

Being liquid at 25°C indicates that the viscosity at 25°C is 10,000 mPa·s or less. Here, the viscosity is measured with a B type viscometer (TVB-10M, manufactured by TOKI SANGYO CO., LTD.), rotor No. 4, 60 rpm, and 60 s.

Nonvolatile refers to one in which the amount of evaporation at 25°C for 6 h measured by the following method (1) is less than 20%.

Method (1): Filter paper having a diameter of 90 mm is placed in a glass petri dish having a diameter of 120 mm, and 1 g of a sample is placed on the filter paper and stored in a room (25°C) at 65% RH. Then, the residue of the sample after 6 h is measured to calculate the amount of evaporation.

The component (C) nonvolatile oil may be one used in ordinary cosmetics, and examples of the nonvolatile oil include linear or branched hydrocarbon oils such as liquid paraffins, liquid isoparaffins, squalane, and squalene; monoester oils such as ethylhexyl para-methoxycinnamate, octyldodecyl myristate, isotridecyl isononanoate, and isononyl isononanoate; diester oils such as neopentyl glycol dicaprate, propylene glycol dicaprate, and diisostearyl malate; triester oils such as glyceryl tri-2-ethylhexanoate and glyceryl tri(caprylate/caprate); tetraester oils such as dipentaerythrityl tetraisostearate; silicone oils such as dimethylpolysiloxane, caprylyl methicone, methylphenylpolysiloxane, methylhydrogenpolysiloxane, and higher alcohol-modified organopolysiloxanes; and fluorine oils such as fluoropolyethers and perfluoroalkyl ether silicones.

Among these, at least one or more selected from the group consisting of linear or branched hydrocarbon oils, monoester oils, diester oils, triester oils, tetraester oils, and silicone oils are preferably included, at least one or more selected from the group consisting of linear or branched hydrocarbon oils, monoester oils, diester oils, and silicone oils are more preferably included, at least one or more selected from the group consisting of branched hydrocarbon oils, monoester oils, diester oils, and silicone oils are further more preferably included, and at least one or more selected from the group consisting of liquid isoparaffins, ethylhexyl para-methoxycinnamate, methylphenylpolysiloxane, caprylyl methicone, neopentyl glycol dicaprate, and propylene glycol dicaprate are even more preferably included, from the viewpoint of reducing the sliding feeling of the water-in-oil emulsion composition on the skin during application and improving the feeling of close adhesion to the skin after application and the pore covering ability.

One or more components (C) can be used, and the content is preferably 0.5% by mass or more, more preferably 2% by mass or more, and further more preferably 7% by mass or more, and preferably 35% by mass or less, more preferably 23% by mass or less, and further more preferably 15% by mass or less in the total composition from the viewpoint of reducing the sliding feeling of the water-in-oil emulsion composition on the skin during application and improving the feeling of close adhesion to the skin after application and the pore covering ability. The content of the component (C) is preferably from 0.5 to 35% by mass, more preferably from 2 to 23% by mass, and further more preferably from 7 to 15% by mass in the total composition.

As the component (D) carbonate, a carbonate of a divalent metal is preferred, and examples thereof include calcium carbonate, magnesium carbonate, and barium carbonate. Among these, at least one or more selected from the group consisting of calcium carbonate and magnesium carbonate are preferably included, and calcium carbonate is more preferred, from the viewpoint of reducing the sliding feeling of the water-in-oil emulsion composition on the skin during application and improving the feeling of close adhesion to the skin after application and the pore covering ability.

In addition, from the viewpoint of reducing the sticky feeling of the skin after application, at least one or more selected from the group consisting of calcium carbonate and magnesium carbonate are preferably included, and magnesium carbonate is more preferred.

Further, from the viewpoint of reducing the sliding feeling of the water-in-oil emulsion composition on the skin during application, improving the feeling of close adhesion to the skin after application and the pore covering ability, and reducing the sticky feeling, calcium carbonate and magnesium carbonate are preferably used in combination.

The component (D) carbonate is used alone. When the carbonate is used as a powder surface treatment agent, or when the carbonate is used as a composite powder raw material, the carbonate is not present alone in the composition, and therefore the effects of the present invention cannot be obtained.

The shape of the component (D) carbonate is not limited and may be any of a spindle shape, a rod shape, a spherical shape, a plate shape, a needle shape, a fiber shape, a petal shape, and the like.

The shape of the component (D) carbonate is preferably a spindle shape, a rod shape, a plate shape, a needle shape, a fiber shape, or a petal shape, more preferably a spindle shape, a rod shape, a plate shape, a needle shape, or a fiber shape, and further more preferably a spindle shape, a rod shape, or a plate shape from the viewpoint of reducing the sliding feeling of the water-in-oil emulsion composition on the skin during application, improving the feeling of close adhesion to the skin after application and the pore covering ability, and reducing the sticky feeling.

The volume average particle diameter of the carbonate is preferably from 0.1 to 50 $\mu$m, more preferably from 1 to 30 $\mu$m, and even more preferably from 6 to 20 $\mu$m from the viewpoint of reducing the sliding feeling of the water-in-oil emulsion composition on the skin during application, improving the feeling of close adhesion to the skin after application and the pore covering ability, and reducing the sticky feeling.

Further, from the viewpoint of reducing the sliding feeling of the water-in-oil emulsion composition on the skin during application, improving the feeling of close adhesion to the skin after application and the pore covering ability, and reducing the sticky feeling, the particle diameter of the long axis with respect to the average thickness (aspect ratio) of the carbonate is preferably 1 or more, more preferably 1.3 or more, and further more preferably 1.6 or more, and preferably 100 or less, more preferably 50 or less, and further more preferably 30 or less. The particle diameter of the long axis with respect to the average thickness (aspect ratio) of the carbonate is preferably from 1 to 100, more preferably from 1.3 to 50, and further more preferably from 1.6 to 30.

Here, the volume average particle diameter is measured by a laser diffraction scattering particle size distribution measuring instrument (manufactured by HORIBA, Ltd., LA-920). In the present invention, the volume average particle diameter is the volume-based average particle diameter and is the 50% median diameter.

The aspect ratio is calculated by the ratio of the volume average particle diameter to the average thickness of the particles and defined by aspect ratio = (volume average particle diameter/average thickness).

The average thickness of the particles is obtained by number-averaging the thickness of from 10 to 50 mother particles measured by observation with a scanning electron microscope or a transmission electron microscope.

As the component (D) carbonate a commercial product can be used. Examples of calcium carbonate include light calcium carbonate (manufactured by Ohmi Chemical Industry Co., Ltd., volume average particle diameter 7.5 $\mu$m, spindle shape, aspect ratio 1.9) and SCS-M5 (manufactured by Sakai Chemical Industry Co., Ltd., volume average particle diameter 5 $\mu$m, spherical shape, aspect ratio 1). Examples of magnesium carbonate include heavy magnesium carbonate (manufactured by Kyowa Chemical Industry Co., Ltd., volume average particle diameter 16 $\mu$m, plate shape, aspect ratio 26).

One or more components (D) can be used, and the content is preferably 0.1% by mass or more, more preferably 0.5% by mass or more, and further more preferably 1.2% by mass or more, and preferably 10% by mass or less, more preferably 8% by mass or less, and further more preferably 5% by mass or less in the total composition from the viewpoint of reducing the sliding feeling of the water-in-oil emulsion composition on the skin during application, improving the feeling of close adhesion to the skin after application and the pore covering ability, and reducing the sticky feeling. The content of the component (D) is preferably from 0.1 to 10% by mass, more preferably from 0.5 to 8% by mass, and further more preferably from 1.2 to 5% by mass in the total composition.

In the present invention, a mass ratio of the component (B) to the component (D), (B)/(D), is preferably 0.007 or more, more preferably 0.01 or more, further more preferably 0.015 or more, and even more preferably 0.02 or more, and preferably 50 or less, more preferably 30 or less, further more preferably 10 or less, and even more preferably 5 or less from the viewpoint of reducing the sliding feeling of the water-in-oil emulsion composition on the skin during application, improving the feeling of close adhesion to the skin after application and the pore covering ability, and reducing the sticky feeling. The mass ratio of the component (B) to the component (D), (B)/(D), is preferably from 0.007 to 50, more preferably from 0.01 to 30, further more preferably from 0.015 to 10, and even more preferably from 0.02 to 5.

In the present invention, a mass ratio of the component (C) to the component (D), (C)/(D), is preferably 0.1 or more, more preferably 0.3 or more, further more preferably 1.5 or more, and even more preferably 2.3 or more, and preferably 100 or less, more preferably 50 or less, further more preferably 20 or less, and even more preferably 10 or less from the viewpoint of reducing the sliding feeling of the water-in-oil emulsion composition on the skin during application, improving the feeling of close adhesion to the skin after application and the pore covering ability, and reducing the sticky feeling. The mass ratio of the component (C) to the component (D), (C)/(D), is preferably from 0.1 to 100, more preferably from 0.3 to 50, further more preferably from 1.5 to 20, and even more preferably from 2.3 to 10.

In the present invention, the content of the component (E) water is preferably 20% by mass or more, more preferably 30% by mass or more, further more preferably 40% by mass or more, and even more preferably 45% by mass or more, and preferably 80% by mass or less, more preferably 70% by mass or less, further more preferably 60% by mass or less, and even more preferably 55% by mass or less in the total composition. The content of the component (E) is preferably from 20 to 80% by mass, more preferably from 30 to 70% by mass, further more preferably from 40 to 60% by mass, and even more preferably from 45 to 55% by mass in the total composition.

The water-in-oil emulsion composition of the present invention can further contain a coloring pigment.

The coloring pigment is one used in ordinary cosmetics, and examples of the coloring pigment include inorganic pigments such as metal oxides such as titanium oxide, zinc oxide, cerium oxide, aluminum oxide, yellow iron oxide, black iron oxide, red oxide, Prussian blue, ultramarine blue, chromium oxide, and chromium hydroxide, metal complexes such as manganese violet and cobalt titanate, and further carbon black; synthetic organic pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, and Blue No. 404; and natural organic dyes such as β-carotin, caramel, and paprika dye, and include composites of these coloring pigments, and composite pigments in which these coloring pigments are combined with pearl pigments. Examples of the pearl pigments to be treated include natural or synthetic inorganic powders such as mica, phlogopite, talc, silica, sericite, glass, kaolin, bismuth oxychloride, cerium oxide, calcium sulfate, barium sulfate, magnesium sulfate, and plate-shaped alumina powders. Specific examples of the composite pigments include titanium oxide-coated mica, iron oxide-coated mica, iron oxide-coated mica titanium, iron oxide-coated synthetic phlogopite, chromium oxide-coated mica titanium, titanium oxide-coated glass powders, iron oxide-coated glass powders, titanium oxide-contained glass powders, and iron oxide-contained glass powders.

Among these, metal oxides are preferred, at least one or more selected from the group consisting of titanium oxide, zinc oxide, yellow iron oxide, black iron oxide, and red oxide are more preferably included, and at least one or more selected from the group consisting of titanium oxide, yellow iron oxide, black iron oxide, and red oxide are further more preferably included.

These coloring pigments can also be used as they are, or they may be also subjected to hydrophobization treatment and used. Examples of the hydrophobization treatment include surface treatment such as fluorine compound treatment, silicone treatment, alkyl treatment, alkylsilane treatment, metallic soap treatment, watersoluble polymer treatment, amino acid treatment, acylated amino acid treatment, lecithin treatment, organotitanate treatment, polyol treatment, acrylic resin treatment, methacrylic resin treatment, and urethane resin treatment.

As the hydrophobization treatment, hydrophobization treatment by at least one or more selected from the group consisting of silicone treatment, amino acid treatment, and acylated amino acid treatment is preferred, and hydrophobization treatment by at least one or more selected from the group consisting of silicone treatment and acylated amino acid treatment is more preferred, from the viewpoint of reducing the sliding feeling of the water-in-oil emulsion composition on the skin during application, improving the feeling of close adhesion to the skin after application and the pore covering ability, and reducing the sticky feeling. From the viewpoint of reducing the sliding feeling of the water-in-oil emulsion composition on the skin during application, improving the feeling of close adhesion to the skin after application and the pore covering ability, and reducing the sticky feeling of the skin, silicone treatment and acylated amino acid treatment are preferably used in combination.

The hydrophobization treatment of the coloring pigment can be performed by an ordinary method.

One or more coloring pigments can be used, and the content is preferably from 1 to 30% by mass, more preferably from 3 to 25% by mass, further more preferably from 5 to 20% by mass, and even more preferably from 7 to 15% by mass in the total composition from the viewpoint of reducing the sliding feeling of the water-in-oil emulsion composition on the skin during application, improving the feeling of close adhesion to the skin after application and the pore covering ability, and reducing the sticky feeling.

The water-in-oil emulsion composition of the present invention can further contain a film-forming agent and can reduce the sliding feeling of the water-in-oil emulsion composition on the skin during application and improve the feeling of close adhesion to the skin after application.

Such a film-forming agent is one used in ordinary cosmetics, and examples of the film-forming agent include fluorine-modified silicone resins, trimethylsiloxysilicate, acrylic silicone resins, and alkylsilsesquioxane resins.

As the fluorine-modified silicone resin, preferred is one having a structure of the following formula (1):

$$R^1{}_g SiO_{(4-g)/2} \qquad (1)$$

wherein $R^1$ is a hydrocarbon group having from 1 to 8 carbon atoms, a phenyl group, a hydroxy group, or the formula -$R^2$-Rf ($R^2$ represents a divalent alkylene group having from 2 to 6 carbon atoms, and Rf represents a perfluoroalkyl group having from 1 to 8 carbon atoms) and is optionally selected from the group consisting of a hydroxy group and a functional group in which the formula -$R^2$-Rf is essential, g is the average number, and $1.0 \le g \le 1.8$, provided that $R^1$ may be the same or different.

As such a fluorine-modified silicone resin, the cosmetic labeling name "trifluoroalkyldimethyltrimethylsiloxysilicate" (INCI name "Trifluoropropyldimethyl/Trimethylsiloxysilicate") is preferred, and a commercial product such as XS66-B8226 (50% by mass decamethylcyclopentasiloxane solution) or XS66-B8636 (50% by mass dimethylpolysiloxane (10 cs) solution) (all manufactured by Momentive Performance Materials Inc.) previously dissolved in a solvent can be used.

As the trimethylsiloxysilicate, a compound having a crosslinked structure having a siloxane structure as the main skeleton and of $[(CH_3)_3SiO_{1/2}]_S[SiO_2]_T$ (S is from 1 to 3, and T is from 0.5 to 8) is preferred.

As the trimethylsiloxysilicate, the cosmetic labeling name "trimethylsiloxysilicate" (INCI name "Trimethylsiloxysilicate") is preferred, and a commercial product such as KF-7312T (60% by mass methyl trimethicone solution), KF-7312L (50% dimethylpolysiloxane (2 cs) solution), KF-7312J (50% by mass decamethylcyclopentasiloxane solution), or KF-7312K (50% by mass dimethylpolysiloxane (6 cs) solution) (all manufactured by Shin-Etsu Chemical Co., Ltd.) previously dissolved in a solvent can be used.

Examples of the acrylic silicone resins include vinyl-based polymers having a carbosiloxane dendrimer structure in the side chain, and acrylic-silicone-based graft copolymers.

Examples of the vinyl-based polymers having the carbosiloxane dendrimer structure in the side chain include those described in JP-A-H11-1530, JP-A-2000-63225, and the like.

As the vinyl-based polymer having the carbosiloxane dendrimer structure in the side chain, a silicone dendrimer-acrylic copolymer is preferred, the cosmetic labeling name "(acrylates/polytrimethylsiloxymethacrylate) copolymer" (INCI name "Acrylates/Polytrimethylsiloxymethacrylate Copolymer") is preferred, and a commercial product such as FA4001CM (30% by mass decamethylcyclopentasiloxane solution), FA4002ID (40% by mass isododecane solution), or DOWSIL FA 4003 DM Silicone Acrylate (40% by mass dimethylpolysiloxane (2 cs) solution) (all manufactured by Dow Toray Co., Ltd.) previously dissolved in a solvent can be used.

Examples of the acrylic-silicone-based graft copolymers include those described in JP-A-2-25411 and the like.

As the acrylic-silicone-based graft copolymer, the cosmetic labeling name "(acrylates/dimethicone) copolymer" (INCI name "Acrylates/Dimethicone Copolymer") is preferred, and a commercial product such as KP545 (30% by mass decamethylcyclopentasiloxane solution), KP549 (40% by mass methyl trimethicone solution), or KP550 (40% by mass isododecane solution) (all manufactured by Shin-Etsu Chemical Co., Ltd.) previously dissolved in a solvent can be used.

As the alkylsilsesquioxane resin, for example, the one described in JP-A-H4-312511 can be used. Specific examples can include a silicone resin obtained by reacting from 5 to 100 parts by mass of a silicone compound of $(R^4{}_3Si)_aZ$, wherein $R^4$ represents a substituted or unsubstituted monovalent hydrocarbon group, a represents 1 or 2, Z represents a hydrogen atom, a hydroxy group, or a hydrolyzable group when a is 1, and Z represents -O-, -N(X)-, or -S- when a is 2; here, X represents a hydrogen atom, a monovalent hydrocarbon group having from 1 to 4 carbon atoms, or $R^4{}_3Si$-, with 100 parts by mass of a silanol group-containing organopolysilsesquioxane comprised of an $R^3SiO_{1.5}$ unit, wherein $R^3$ represents a substituted or unsubstituted monovalent hydrocarbon group, such as a silicone resin containing from 50 to 99 mol% of the $R^3SiO_{1.5}$ unit and from 1 to 50 mol% of the $R^4{}_3SiO_{0.5}$ unit.

As the alkylsilsesquioxane resin, the cosmetic labeling name "polypropylsilsesquioxanecyclopentasiloxane" (INCI name "POLYPROPYLSILSESQUIOXANECYCLOPENTASILOXANE") is preferred. As the commercial product, for example, 670FLUID (manufactured by Dow Toray Co., Ltd.), which is polypropylsilsesquioxane, or DOWSIL MQ-1640 FLAKE RESIN (mixture of 37% by mass of polypropylsilsesquioxane and 63% by mass of trimethylsiloxysilicate) (manufactured by Dow Toray Co., Ltd.), which is a mixture of polypropylsilsesquioxane and trimethylsiloxysilicate, can be used.

The film-forming agent preferably contains at least one or more selected from the group consisting of trimethylsiloxysilicate, acrylic silicone resins, and alkylsilsesquioxane resins and is more preferably an acrylic silicone resin, further more preferably a vinyl-based polymer having a carbosiloxane dendrimer structure in the side chain, from the viewpoint of reducing the sliding feeling of the water-in-oil emulsion composition on the skin during application and improving the feeling of close adhesion to the skin after application.

One or more film-forming agents can be used, and the content is preferably from 0.1 to 10% by mass, more preferably from 0.5 to 7% by mass, and further more preferably from 1.5 to 4% by mass in the total composition from the viewpoint of reducing the sliding feeling of the water-in-oil emulsion composition on the skin during application and improving the feeling of close adhesion to the skin after application.

The water-in-oil emulsion composition of the present invention can further contain a volatile oil and can reduce the sliding feeling of the water-in-oil emulsion composition on the skin during application.

Here, being volatile refers to one in which the amount of evaporation at 25°C for 6 h measured by the following method (1) is 20% or more.

Method (1): Filter paper having a diameter of 90 mm is placed in a glass petri dish having a diameter of 120 mm, and 1 g of a sample is placed on the filter paper and stored in a room (25°C) at 65% RH. Then, the residue of the sample after 6 h is measured to calculate the amount of evaporation.

The volatile oil may be any used in ordinary cosmetics, and examples of the volatile oil include silicone oils, hydrocarbon oils, and ether oils.

Examples of the silicone oils include chain dimethylpolysiloxane and cyclic dimethylpolysiloxane. The chain dimethylpolysiloxane may be either linear or branched. Examples of the linear dimethylpolysiloxane include dimethylpolysiloxane (1.5 cs) and dimethylpolysiloxane (2 cs). Examples of the branched dimethylpolysiloxane include methyl trimethicone, tris(trimethylsilyl)methylsilane, and tetrakis(trimethylsilyl)silane. Examples of the cyclic dimethylpolysiloxane include octamethylcyclotetrasiloxane, decamethylcyclopentasiloxane, and dodecamethylcyclohexasiloxane.

Examples of the hydrocarbon oils include isododecane, isotridecane, isohexadecane, light isoparaffins, and light liquid isoparaffins, and examples of the ether oils include ethyl perfluorobutyl ether.

Among these, at least one or more selected from the group consisting of silicone oils and hydrocarbon oils are preferably included, at least one or more selected from the group consisting of dimethylpolysiloxane (2 cs), decamethylcyclopentasiloxane, isododecane, and light liquid isoparaffins are more preferably included, and at least one or more selected from the group consisting of dimethylpolysiloxane (2 cs), decamethylcyclopentasiloxane, and light liquid isoparaffins are even more preferably included, from the viewpoint of reducing the sliding feeling of the water-in-oil emulsion composition on the skin during application.

One or more volatile oils can be used, and the content is preferably from 1 to 30% by mass, more preferably from 3 to 20% by mass, and further more preferably from 5 to 15% by mass in the total composition from the viewpoint of reducing the sliding feeling of the water-in-oil emulsion composition on the skin during application.

In the water-in-oil emulsion composition of the present invention, from the viewpoint of environmental consideration and an excellent use impression, the content of an organic resin powder is preferably 1% by mass or less, more preferably 0.1% by mass or less, in the total composition, and the water-in-oil emulsion composition of the present invention further more preferably contains substantially no organic resin powder.

The content of a spherical organic resin powder is preferably 1% by mass or less, more preferably 0.1% by mass or less, in the total composition, and the water-in-oil emulsion composition of the present invention further more preferably contains substantially no spherical organic resin powder.

Examples of the organic resin powder include crosslinked or non-crosslinked organic resin powders such as powders of one or more polymers or copolymers selected from the group consisting of polyamide resins, nylon resins, polyester resins, polyethylene resins, polytetrafluoroethylene resins, polypropylene resins, polystyrene resins, benzoguanamine resins, polymethylbenzoguanamine resins, polyurethane resins, vinyl resins, fluororesins, acrylic resins, and melamine resins; butyl acrylate-vinyl acetate copolymers, styrene-acrylic acid copolymers, silicone resins, and divinylbenzene-styrene copolymers.

The water-in-oil emulsion composition of the present invention can contain, in addition to the components, components usually used in cosmetics, for example, an extender pigment such as talc, a powder other than the above, a polymer compound, an antioxidant, a perfume, a preservative, a pH adjusting agent, a blood circulation promoter, a cold sense agent, an antiperspirant, a disinfectant, a skin activator, a moisturizer, and a refrigerant.

The water-in-oil emulsion composition of the present invention can be produced in accordance with an ordinary method, and can be in a liquid, emulsion, pasty, creamy, gel, or solid form or the like and is preferably pasty, creamy, or solid.

The water-in-oil emulsion composition of the present invention can be applied as water-in-oil emulsion cosmetics and can be applied as makeup cosmetics such as cosmetic bases, foundations, concealers, blushers, eye shadows, mascaras, eyeliners, eyebrow pencils, overcoat agents, and lipsticks; ultraviolet protecting cosmetics such as sunscreen emulsions and sunscreen creams; and the like. The water-in-oil emulsion composition of the present invention is preferred as makeup cosmetics.

Regarding the embodiment, the present invention further discloses the following compositions.

<1> A water-in-oil emulsion composition comprising the following components (A), (B), (C), (D), and (E):

(A) a lipophilic nonionic surfactant;
(B) an oil agent which is solid or semisolid at 25°C;
(C) a nonvolatile oil which is liquid at 25°C;
(D) a carbonate; and

(E) water.

<2> The water-in-oil emulsion composition according to <1>, wherein the component (A) preferably has an HLB of from 1 to 8, more preferably from 2 to 7, and further more preferably from 3 to 6.

<3> The water-in-oil emulsion composition according to <1> or <2>, wherein the component (A) preferably comprises at least one or more selected from the group consisting of sorbitan fatty acid esters and silicone-based surfactants, more preferably at least one or more selected from the group consisting of sorbitan fatty acid esters and polyether-modified silicones, further more preferably at least one or more selected from the group consisting of sorbitan monoisostearate, sorbitan monooleate, sorbitan sesquistearate, and polyether-modified silicones, and even more preferably at least one or more selected from the group consisting of sorbitan monoisostearate and polyoxyethylene-methylpolysiloxane copolymers.

<4> The water-in-oil emulsion composition according to any one of <1> to <3>, wherein in the component (A), a sorbitan fatty acid ester and a silicone-based surfactant are preferably used in combination.

<5> The water-in-oil emulsion composition according to any one of <1> to <4>, wherein a content of the component (A) is preferably 1% by mass or more, more preferably 1.5% by mass or more, and further more preferably 2% by mass or more, and preferably 8% by mass or less, more preferably 6.5% by mass or less, and further more preferably 5.5% by mass or less in a total composition.

<6> The water-in-oil emulsion composition according to any one of <1> to <5>, wherein the component (B) preferably comprises at least one or more selected from the group consisting of vegetable waxes, cholesterol derivatives, phytosterol derivatives, triglycerides, lanolin, Vaseline, and dimer dilinoleic acid hydrogenated castor oil, more preferably at least one or more selected from the group consisting of vegetable waxes, cholesterol derivatives, phytosterol derivatives, triglycerides, lanolin, and Vaseline, further more preferably at least one or more selected from the group consisting of vegetable waxes, phytosterol derivatives, and triglycerides, even more preferably at least one or more selected from the group consisting of vegetable waxes and triglycerides, and especially preferably a triglyceride.

<7> The water-in-oil emulsion composition according to any one of <1> to <5>, wherein the component (B) preferably comprises at least one or more selected from the group consisting of shea butter, (phytosteryl/isostearyl/cetyl/stearyl/behenyl) dimer dilinoleate, and glyceryl tribehenate.

<8> The water-in-oil emulsion composition according to any one of <1> to <7>, wherein a content of the component (B) is preferably 0.01% by mass or more, more preferably 0.03% by mass or more, further more preferably 0.07% by mass or more, and preferably 8% by mass or less, more preferably 5% by mass or less, and further more preferably 3% by mass or less in the total composition.

<9> The water-in-oil emulsion composition according to any one of <1> to <8>, wherein the component (C) preferably comprises at least one or more selected from the group consisting of linear or branched hydrocarbon oils, monoester oils, diester oils, triester oils, tetraester oils, and silicone oils, more preferably at least one or more selected from the group consisting of linear or branched hydrocarbon oils, monoester oils, diester oils, and silicone oils, further more preferably at least one or more selected from the group consisting of branched hydrocarbon oils, monoester oils, diester oils, and silicone oils, and even more preferably at least one or more selected from the group consisting of liquid isoparaffins, ethylhexyl para-methoxycinnamate, methylphenylpolysiloxane, caprylyl methicone, neopentyl glycol dicaprate, and propylene glycol dicaprate.

<10> The water-in-oil emulsion composition according to any one of <1> to <9>, wherein a content of the component (C) is preferably 0.5% by mass or more, more preferably 2% by mass or more, and further more preferably 7% by mass or more, and preferably 35% by mass or less, more preferably 23% by mass or less, and further more preferably 15% by mass or less in the total composition.

<11> The water-in-oil emulsion composition according to any one of <1> to <10>, wherein the component (D) preferably comprises at least one or more selected from the group consisting of calcium carbonate and magnesium carbonate.

<12> The water-in-oil emulsion composition according to any one of <1> to <11>, wherein in the component (D), calcium carbonate and magnesium carbonate are preferably used in combination.

<13> The water-in-oil emulsion composition according to any one of <1> to <12>, wherein a content of the component (D) is preferably 0.1% by mass or more, more preferably 0.5% by mass or more, and further more preferably 1.2% by mass or more, and preferably 10% by mass or less, more preferably 8% by mass or less, and further more preferably 5% by mass or less in the total composition.

<14> The water-in-oil emulsion composition according to any one of <1> to <13>, wherein a mass ratio of the component (B) to the component (D), (B)/(D), is preferably 0.007 or more, more preferably 0.01 or more, further more preferably 0.015 or more, and even more preferably 0.02 or more, and preferably 50 or less, more preferably 30 or less, further more preferably 10 or less, and even more preferably 5 or less.

<15> The water-in-oil emulsion composition according to any one of <1> to <14>, wherein a mass ratio of the component (C) to the component (D), (C)/(D), is preferably 0.1 or more, more preferably 0.3 or more, further more preferably 1.5 or more, and even more preferably 2.3 or more, and preferably 100 or less, more preferably 50 or less, further more preferably 20 or less, and even more preferably 10 or less.

<16> The water-in-oil emulsion composition according to any one of <1> to <15>, wherein a content of the component (E) water is preferably 20% by mass or more, more preferably 30% by mass or more, further more preferably 40% by mass or more, and even more preferably 45% by mass or more, and preferably 80% by mass or less, more preferably 70% by mass or less, further more preferably 60% by mass or less, and even more preferably 55% by mass or less in the total composition.

<17> The water-in-oil emulsion composition according to any one of <1> to <16>, preferably further comprising a coloring pigment, wherein the coloring pigment is preferably a metal oxide and more preferably comprises at least one or more selected from the group consisting of titanium oxide, zinc oxide, yellow iron oxide, black iron oxide, and red oxide, further more preferably at least one or more selected from the group consisting of titanium oxide, yellow iron oxide, black iron oxide, and red oxide.

<18> The water-in-oil emulsion composition according to <17>, wherein the coloring pigment is preferably subjected to hydrophobization treatment, more preferably hydrophobization treatment by at least one or more selected from the group consisting of silicone treatment, amino acid treatment, and acylated amino acid treatment, and further more preferably hydrophobization treatment by at least one or more selected from the group consisting of silicone treatment and acylated amino acid treatment.

<19> The water-in-oil emulsion composition according to <17> or <18>, wherein a content of the coloring pigment is preferably from 1 to 30% by mass, more preferably from 3 to 25% by mass, further more preferably from 5 to 20% by mass, and even more preferably from 7 to 15% by mass in the total composition.

<20> The water-in-oil emulsion composition according to any one of <1> to <19>, preferably further comprising a film-forming agent, wherein the film-forming agent is preferably a fluorine-modified silicone resin, trimethylsiloxysilicate, an acrylic silicone resin, or an alkylsilsesquioxane resin, more preferably comprises at least one or more selected from the group consisting of trimethylsiloxysilicate, acrylic silicone resins, and alkylsilsesquioxane resins, and is further more preferably an acrylic silicone resin, even more preferably a vinyl-based polymer having a carbosiloxane dendrimer structure in its side chain.

<21> The water-in-oil emulsion composition according to <20>, wherein a content of the film-forming agent is preferably from 0.1 to 10% by mass, more preferably from 0.5 to 7% by mass, and further more preferably from 1.5 to 4% by mass in the total composition.

<22> The water-in-oil emulsion composition according to any one of <1> to <21>, preferably further comprising a volatile oil, wherein the volatile oil preferably comprises at least one or more selected from the group consisting of silicone oils and hydrocarbon oils, more preferably at least one or more selected from the group consisting of dimethylpolysiloxane (2 cs), decamethylcyclopentasiloxane, isododecane, and light liquid isoparaffins, and even more preferably at least one or more selected from the group consisting of dimethylpolysiloxane (2 cs), decamethylcyclopentasiloxane, and light liquid isoparaffins.

<23> The water-in-oil emulsion composition according to <22>, wherein a content of the volatile oil is preferably from 1 to 30% by mass, more preferably from 3 to 20% by mass, and further more preferably from 5 to 15% by mass in the total composition.

<24> The water-in-oil emulsion composition according to any one of <1> to <23>, wherein a content of an organic resin powder is preferably 1% by mass or less, more preferably 0.1% by mass or less, in the total composition, the water-in-oil emulsion composition further more preferably comprising substantially no organic resin powder.

<25> The water-in-oil emulsion composition according to any one of <1> to <24>, wherein a content of a spherical organic resin powder is preferably 1% by mass or less, more preferably 0.1% by mass or less, in the total composition, the water-in-oil emulsion composition further more preferably comprising substantially no spherical organic resin powder.

<26> The water-in-oil emulsion composition according to any one of <1> to <25>, being a water-in-oil emulsion cosmetic.

<27> A water-in-oil emulsion composition comprising the following components (A), (B), (C), (D), and (E):

(A) from 1 to 8% by mass of at least one or more lipophilic nonionic surfactants selected from the group consisting of sorbitan fatty acid esters and silicone-based surfactants;
(B) from 0.01 to 8% by mass of an oil agent which is solid or semisolid at 25°C;
(C) from 0.5 to 35% by mass of a nonvolatile oil which is liquid at 25°C;
(D) from 0.1 to 10% by mass of at least one or more carbonates selected from the group consisting of calcium carbonate and magnesium carbonate; and
(E) water.

<28> A water-in-oil emulsion composition comprising the following components (A), (B), (C), (D), and (E):

(A) from 1 to 8% by mass of at least one or more lipophilic nonionic surfactants selected from the group consisting of sorbitan fatty acid esters and silicone-based surfactants;
(B) from 0.01 to 8% by mass of an oil agent which is solid or semisolid at 25°C;
(C) from 0.5 to 35% by mass of a nonvolatile oil which is liquid at 25°C;
(D) from 0.1 to 10% by mass of at least one or more carbonates selected from the group consisting of calcium carbonate and magnesium carbonate; and
(E) water, and

from 1 to 30% by mass of a coloring pigment.
<29> A water-in-oil emulsion composition comprising the following components (A), (B), (C), (D), and (E):

(A) from 2 to 5.5% by mass of at least one or more lipophilic nonionic surfactants selected from the group consisting of sorbitan fatty acid esters and silicone-based surfactants;
(B) from 0.07 to 3% by mass of an oil agent which is solid or semisolid at 25°C;
(C) from 0.5 to 35% by mass of a nonvolatile oil which is liquid at 25°C;
(D) from 1.2 to 5% by mass of at least one or more carbonates selected from the group consisting of calcium carbonate and magnesium carbonate; and
(E) water.

<30> A water-in-oil emulsion composition comprising the following components (A), (B), (C), (D), and (E):

(A) from 1 to 8% by mass of at least one or more lipophilic nonionic surfactants selected from the group consisting of sorbitan fatty acid esters and silicone-based surfactants;
(B) from 0.01 to 8% by mass of at least one or more oil agents which are solid or semisolid at 25°C selected from the group consisting of vegetable waxes, phytosterol derivatives, and triglycerides;
(C) from 0.5 to 35% by mass of a nonvolatile oil which is liquid at 25°C;
(D) from 0.1 to 10% by mass of at least one or more carbonates selected from the group consisting of calcium carbonate and magnesium carbonate; and
(E) water.

<31> A water-in-oil emulsion composition comprising the following components (A), (B), (C), (D), and (E):

(A) from 2 to 5.5% by mass of at least one or more lipophilic nonionic surfactants selected from the group consisting of sorbitan fatty acid esters and silicone-based surfactants;
(B) from 0.07 to 3% by mass of at least one or more oil agents which are solid or semisolid at 25°C selected from the group consisting of vegetable waxes, phytosterol derivatives, and triglycerides;
(C) from 0.5 to 35% by mass of a nonvolatile oil which is liquid at 25°C;
(D) from 1.2 to 5% by mass of at least one or more carbonates selected from the group consisting of calcium carbonate and magnesium carbonate; and
(E) water.

<32> A water-in-oil emulsion composition comprising the following components (A), (B), (C), (D), and (E):

(A) from 2 to 5.5% by mass of at least one or more lipophilic nonionic surfactants selected from the group consisting of sorbitan fatty acid esters and silicone-based surfactants;
(B) from 0.07 to 3% by mass of at least one or more oil agents which are solid or semisolid at 25°C selected from the group consisting of vegetable waxes, phytosterol derivatives, and triglycerides;
(C) from 0.5 to 35% by mass of a nonvolatile oil which is liquid at 25°C;
(D) from 1.2 to 5% by mass of at least one or more carbonates selected from the group consisting of calcium carbonate and magnesium carbonate; and
(E) water, wherein

a mass ratio of the component (B) to the component (D), (B)/(D), is from 0.02 to 5.
<33> Use of the water-in-oil emulsion composition according to any one of <1> to <32> as a water-in-oil emulsion cosmetic.
<34> Use of the water-in-oil emulsion composition according to any one of <1> to <32> as a makeup cosmetic.
<35> Use for makeup of the water-in-oil emulsion composition according to any one of <1> to <32>, which is applied to a skin, preferably a face.

<36> Use of the water-in-oil emulsion composition according to any one of <1> to <32> for the production of the water-in-oil emulsion cosmetic.

Examples

Examples 1 to 13 and Comparative Example 1

Water-in-oil emulsion cosmetics having the compositions shown in Table 1 and Table 2 were produced, and the absence of a sliding feeling during application, the feeling of close adhesion to the skin after application, the pore covering ability, and the absence of stickiness of the skin after application were evaluated. The results are shown together in Table 1 and Table 2.

(Production Method)

Components (A), (B), and (C) and other oily components were heated with stirring at 80°C to prepare an oil phase mixture, and a previously pulverized and mixed powder phase was added to the oil phase mixture and mixed using a homomixer. Subsequently, a water phase heated to 80°C was added, mixed, and then cooled to room temperature to obtain a water-in-oil emulsion cosmetic.

(Evaluation Methods)

(1) Absence of Sliding Feeling during Application:

When three expert evaluators took 0.3 g of each water-in-oil emulsion cosmetic on a finger and applied it to the face, they evaluated the absence of a sliding feeling during application by the following criteria. The result is shown by the total points of the three expert evaluators.
The sliding feeling indicates that when the water-in-oil emulsion cosmetic is taken on a fingertip or the like and applied to the face, there is a feeling that the water-in-oil emulsion cosmetic spreads for a long period of time on the skin as if a liquid oil agent were spread, and there is no actual feeling that the water-in-oil emulsion cosmetic spreads while adhering to the skin.

    5; The evaluator clearly feels no sliding feeling.
    4; The evaluator hardly feels a sliding feeling.
    3; The evaluator does not feel a sliding feeling very much.
    2; The evaluator somewhat feels a sliding feeling.
    1; The evaluator clearly feels a sliding feeling.

(2) Feeling of Close Adhesion to Skin after Application:

Three expert evaluators took 0.3 g of each water-in-oil emulsion cosmetic on a finger, applied it to the face, spread it, and then evaluated the feeling of close adhesion to the skin after application by the following criteria. The result is shown by the total points of the three expert evaluators.
The feeling of close adhesion indicates that the water-in-oil emulsion composition attached to the skin after application follows the movement of the skin but does not come off the surface of the skin even if the skin of the face is moved by a change in expression, or the like; and the water-in-oil emulsion composition does not gather partially in the form of large wrinkles, small wrinkles, and the like; and the water-in-oil emulsion composition adheres firmly.

    5; The evaluator clearly feels a feeling of close adhesion.
    4; The evaluator considerably feels a feeling of close adhesion.
    3; The evaluator feels a feeling of close adhesion.
    2; The evaluator does not feel a feeling of close adhesion much.
    1; The evaluator clearly feels no feeling of close adhesion.

(3) Pore Covering Ability:

Three expert evaluators took 0.3 g of each water-in-oil emulsion cosmetic on a finger, applied it to the face, spread it, and then evaluated the pore covering ability by the following criteria. The result is shown by the total points of the three expert evaluators.

5; The evaluator feels that the pores are clearly hidden.
4; The evaluator feels that the pores are hidden.
3; The evaluator feels that the pores are substantially hidden.
2; The evaluator feels that the pores are not hidden so much.
1; The evaluator feels that the pores are clearly not hidden.

(4) Absence of Stickiness of Skin after Application:

Three expert evaluators took 0.3 g of each water-in-oil emulsion cosmetic on a finger, applied it to the face, then touched the skin after application, and evaluated the absence of stickiness by the following criteria. The result is shown by the total points of the three expert evaluators.

5; The evaluator clearly feels no stickiness.
4; The evaluator hardly feels stickiness.
3; The evaluator does not feel stickiness very much.
2; The evaluator somewhat feels stickiness.
1; The evaluator clearly feels stickiness.

[Table 1]

| Component (% by mass) | | | Example 1 | Example 2 | Example 3 | Example 4 | Example 5 | Example 6 | Example 7 | Example 8 | Comparative Example 1 |
|---|---|---|---|---|---|---|---|---|---|---|---|
| Oil phase | (A) | Sorbitan monoisostearate *1 | 0.5 | 0.5 | 0.5 | 0.5 | 0.5 | 0.5 | 0.5 | 0.5 | 0.5 |
| | (A) | Polyoxyethylene-methylpolysiloxane copolymer *2 | 4 | 4 | 4 | 2 | 2 | 4 | 4 | 4 | 4 |
| | (B) | Glyceryl tribehenate *3 | 0.1 | 0.1 | 0.1 | 2 | 2 | | | 0.1 | 0.1 |
| | (8) | Shea butter | | | | | | 0.2 | | | |
| | (8) | (Phytosteryl/isostearyl/cetyl/stearyl/behenyl)dimer dilinoleate *4 | | | | | | | 0.5 | | |
| | (C) | Liquid isoparaffin | 0.5 | 0.5 | 0.5 | 1 | 1 | 0.5 | 0.5 | 0.5 | 0.5 |
| | (C) | 2-Ethylhexyl para-methoxycinnamate *5 | | | | 4 | 4 | | | | |
| | (C) | Methylphenylpolysiloxane *6 | 4 | 4 | 4 | 3 | 3 | 4 | 4 | 4 | 4 |
| | (C) | Caprylyl methicone | 5 | 5 | 5 | 4 | 4 | 5 | 5 | 5 | 5 |
| | (C) | Propylene glycol dicaprate | 1.5 | 1.5 | 1.5 | | | | | 1.5 | 1.5 |
| | (C) | Neopentyl glycol dicaprate | | | | 1 | 1 | 2.5 | 2 | | |

(continued)

| Component (% by mass) | | Example 1 | Example 2 | Example 3 | Example 4 | Example 5 | Example 6 | Example 7 | Example 8 | Comparative Example 1 |
|---|---|---|---|---|---|---|---|---|---|---|
| Dimethylpolysiloxane (2cs) *7 | | 5.5 | 5.5 | 5.5 | | | 5.5 | 5.7 | 5.5 | 5.5 |
| Decamethylcyclopentasiloxane | | | | | 5 | 7 | | | | |
| Light liquid isoparaffin *8 | | 1.5 | 1.5 | 1.5 | | | 1.5 | 1.5 | 1.5 | 1.5 |
| (Acrylates/polytrimethylsiloxymethacrylate) copolymer-dimethicone mixture (40% by mass dimethylpolysiloxane (2 cs) solution) *9 | | 7 | 7 | 7 | 7 | 7 | 7 | 7 | 7 | 7 |
| Glycerin | | 1 | 1 | 1 | | | 1 | 1 | 1 | 1 |
| Dipropylene glycol | | 4 | 4 | 4 | 4 | 4 | 4 | 4 | 4 | 4 |
| Diglycerin | | | | | 1 | 1 | | | | |
| Sodium chloride | | 0.5 | 0.5 | 0.5 | 0.5 | 0.5 | 0.5 | 0.5 | 0.5 | 0.5 |
| Phenoxyethanol | | 0.5 | 0.5 | 0.5 | 0.5 | 0.5 | 0.5 | 0.5 | 0.5 | 0.5 |
| Purified water | (E) | 47.9 | 47.9 | 47.9 | 48.6 | 48.6 | 47.9 | 47.9 | 47.9 | 47.9 |
| Water phase / Dimethicone-disodium stearoyl glutamate-composite-treated titanium oxide *10 | | 10 | 10 | 10 | 7 | 7 | 7 | 7 | 10 | 10 |

| Component (% by mass) | | | Example 1 | Example 2 | Example 3 | Example 4 | Example 5 | Example 6 | Example 7 | Example 8 | Comparative Example 1 |
|---|---|---|---|---|---|---|---|---|---|---|---|
| Powder phase | Hydrogen dimethicone-treated titanium oxide | *11 | | | | 0.5 | 0.5 | | | | |
| | Dimethicone-disodium stearoyl qlutamate-composite-treated red oxide | '12 | 0.3 | 0.3 | 0.3 | 0.2 | 0.2 | 0.2 | 0.2 | 0.3 | 0.3 |
| | Dimethicone-disodium stearoyl glutamate-composite-treated yellow iron oxide | *13 | 1.3 | 1.3 | 1.3 | 0.9 | 0.9 | 0.9 | 0.9 | 1.3 | 1.3 |
| | Dimethicone-disodium stearoyl glutamate-composite-treated black iron oxide | *14 | 0.15 | 0.15 | 0.15 | 0.12 | 0.12 | 0.12 | 0.12 | 0.15 | 0.15 |
| | Dimethicone-disodium stearoyl clutamate-composite-treated talc | *15 | 2.25 | 2.25 | 2.25 | 2.18 | 2.18 | 2.18 | 2.18 | 2.25 | 2.25 |
| | Dimethicone-treated silica | *16 | 0.5 | 0.5 | 0.5 | 0.5 | 0.5 | 0.5 | 0.5 | 0.5 | 2.5 |

(continued)

| Component (% by mass) | | | Example 1 | Example 2 | Example 3 | Example 4 | Example 5 | Example 6 | Example 7 | Example 8 | Comparative Example 1 |
|---|---|---|---|---|---|---|---|---|---|---|---|
| (D) | Calcium carbonate | *17 | 0.5 | 2 | | 3 | 1 | 3 | 3 | | |
| (D) | Calcium carbonate | *18 | | | | | | | | 2 | |
| (D) | Magnesium carbonate | *19 | 1.5 | | 2 | 1.5 | 1.5 | 1.5 | 1.5 | | |
| | Total | | 100.00 | 100.00 | 100.00 | 100.00 | 10000 | 100.00 | 100.00 | 100.00 | 10000 |
| | (A) Total amount | | 4.50 | 4.50 | 4.50 | 2.50 | 2.50 | 4.50 | 4.50 | 4.50 | 4.50 |
| | (B) Total amount | | 0.10 | 0.10 | 0.10 | 2.00 | 2.00 | 0.20 | 0.50 | 0.10 | 0.10 |
| | (C) Total amount | | 1100 | 1100 | 1100 | 13.00 | 13.00 | 1200 | 11.50 | 1100 | 1100 |
| | (D) Total amount | | 2.00 | 2.00 | 2.00 | 4.50 | 2.50 | 4.50 | 4.50 | 2.00 | - |
| | (B)/D) | | 0.05 | 0.05 | 0.05 | 0.44 | 0.80 | 0.04 | 0.11 | 0.05 | - |
| | (C)/(D) | | 5.50 | 5.50 | 5.50 | 2.89 | 5.20 | 2.67 | 2.56 | 5.50 | - |
| | Absence of sliding feeling during application | | 15 | 15 | 13 | 15 | 14 | 14 | 13 | 12 | 3 |
| | Feeling of close adhesion to skin after application | | 15 | 15 | 12 | 15 | 14 | 13 | 14 | 12 | 10 |
| | Pore covering ability | | 14 | 14 | 13 | 13 | 13 | 13 | 10 | 13 | 5 |
| | Absence of stickiness of skin after application | | 14 | 13 | 15 | 14 | 15 | 13 | 10 | 14 | 6 |

EP 4 230 190 A1

[Table 2]

| Component (% by mass) | | | | Example 9 | Example 10 | Example 11 | Example 12 | Example 13 |
|---|---|---|---|---|---|---|---|---|
| Oil phase | (A) | Sorbitan monoisostearate | *1 | 0.5 | 0.5 | 0.5 | 0.5 | 0.5 |
| | (A) | Polyoxyethylene-methylpolysiloxane copolymer | *2 | 4 | 4 | 4 | 4 | 4 |
| | (B) | Glyceryl tribehenate | *3 | 0.05 | 2.5 | 0.1 | 0.1 | 0.1 |
| | (C) | Liquid isoparaffin | | 0.5 | 0.5 | 0.5 | 0.5 | 0.5 |
| | (C) | Methylphenylpolysiloxane | *6 | 4 | 4 | 4 | 4 | 4 |
| | (C) | Caprylyl methicone | | 5 | 5 | 5 | 5 | 5 |
| | (C) | Propylene glycol dicaprate | | 1.5 | 1.5 | 1.5 | 1.5 | 1.5 |
| | | Dimethylpolysiloxane (2cs) | *7 | 5.5 | 5.5 | 5.5 | 5.5 | 12.5 |
| | | Light liquid isoparaffin | *8 | 1.5 | 1.5 | 1.5 | 1.5 | 1.5 |
| | | (Acrylates/polytrimethylsiloxymethacrylate) copolymer-dimethicone mixture (40% by mass dimethylpolysiloxane (2 cs) solution) | *9 | 7 | 7 | 7 | 7 | - |
| Water phase | | Glycerin | | 1 | 1 | 1 | 1 | 1 |
| | | Dipropylene glycol | | 4 | 4 | 4 | 4 | 4 |
| | | Sodium chloride | | 0.5 | 0.5 | 0.5 | 0.5 | 0.5 |
| | | Phenoxyethanol | | 0.5 | 0.5 | 0.5 | 0.5 | 0.5 |
| | (E) | Purified water | | 47.95 | 45.5 | 49.1 | 45.9 | 45.9 |

EP 4 230 190 A1

18

(continued)

| Component (% by mass) | | | | Example 9 | Example 10 | Example 11 | Example 12 | Example 13 |
|---|---|---|---|---|---|---|---|---|
| Powder phase | | Dimethicone-disodium stearoyl glutamate-composite-treated titanium oxide | *10 | 10 | 10 | 10 | 10 | 10 |
| | | Dimethicone-disodium stearoyl glutamate-composite-treated red oxide | *12 | 0.3 | 0.3 | 0.3 | 0.3 | 0.3 |
| | | Dimethicone-disodium stearoyl glutamate-composite-treated yellow iron oxide | *13 | 1.3 | 1.3 | 1.3 | 1.3 | 1.3 |
| | | Dimethicone-disodium stearoyl glutamate-composite-treated black iron oxide | *14 | 0.15 | 0.15 | 0.15 | 0.15 | 0.15 |
| | | Dimethicone-disodium stearoyl glutamate-composite-treated talc | *15 | 2.25 | 2.25 | 2.25 | 2.25 | 2.25 |
| | | Dimethicone-treated silica | *16 | 0.5 | 0.5 | 0.5 | 0.5 | 0.5 |
| | (D) | Calcium carbonate | *17 | 0.5 | 0.5 | 0.2 | 1 | 1 |
| | (D) | Magnesium carbonate | *19 | 1.5 | 1.5 | 0.6 | 3 | 3 |
| | | Total | | 100.00 | 100.00 | 100.00 | 100.00 | 10000 |
| | | (A) Total amount | | 4.50 | 4.50 | 4.50 | 4.50 | 4.50 |
| | | (B) Total amount | | 0.05 | 2.50 | 0.10 | 0.10 | 0.10 |
| | | (C) Total amount | | 1100 | 1100 | 1100 | 1100 | 1100 |
| | | (D) Total amount | | 2.00 | 2.00 | 0.80 | 4.00 | 4.00 |
| | | (B)/(D) | | 0.03 | 1.25 | 0.13 | 0.03 | 0.03 |
| | | (C)/(D) | | 5.50 | 5.50 | 13.75 | 2.75 | 2.75 |
| | | Absence of sliding feeling during application | | 12 | 15 | 13 | 14 | 14 |
| | | Feeling of close adhesion to skin after application | | 11 | 15 | 10 | 14 | 11 |
| | | Pore covering ability | | 12 | 13 | 11 | 14 | 14 |

(continued)

| Component (% by mass) | Example 9 | Example 10 | Example 11 | Example 12 | Example 13 |
|---|---|---|---|---|---|
| Absence of stickiness of skin after application | 14 | 14 | 13 | 15 | 14 |

*1: sorbitan monoisostearate: manufactured by Croda, Span 120, *2: polyoxyethylene-methylpolysiloxane copolymer: manufactured by Shin-Etsu Chemical Co., Ltd., KF-6017, *3: glyceryl tribehenate: manufactured by Croda, Syncrowax HR-C, *4: (phytosteryl/isostearyl/cetyl/stearyl/behenyl) dimer dilinoleate: manufactured by NIPPON FINE CHEMICAL CO., LTD., PLANDOOL-H, *5: 2-ethylhexyl para-methoxycinnamate: manufactured by BASF, Uvinul MC80, *6: methylphenylpolysiloxane: manufactured by Dow Toray Co., Ltd., DOWSIL FZ-209, *7: dimethylpolysiloxane (2 cs): manufactured by Shin-Etsu Chemical Co., Ltd., KF-96L-2 cs, *8: light liquid isoparaffin: manufactured by NOF CORPORATION, PARLEAM 4, *9: (acrylates/polytrimethylsiloxymethacrylate) copolymer-dimethicone mixture: manufactured by Dow Toray Co., Ltd., DOWSIL FA 4003 DM Silicone Acrylate (40% by mass dimethylpolysiloxane (2 cs) solution), *10: dimethicone-disodium stearoyl glutamate-composite-treated titanium oxide: manufactured by Miyoshi Kasei, Inc., SA/NAI-TR-10 MIBRID COLOR POWDER, *11: hydrogen dimethicone-treated titanium oxide: manufactured by DAITO KASEI KOGYO CO., LTD., SI01-2 TIO2 MT-500SA, *12: dimethicone-disodium stearoyl glutamate-composite-treated red oxide: manufactured by Miyoshi Kasei, Inc., SA/NAI-R-10 MIBRID COLOR POWDER, *13: dimethicone-disodium stearoyl glutamate-composite-treated yellow iron oxide: manufactured by Miyoshi Kasei, Inc., SA/NAI-Y-10 MIBRID COLOR POWDER, *14: dimethicone-disodium stearoyl glutamate-composite-treated black iron oxide: manufactured by Miyoshi Kasei, Inc., SA/NAI-B-10 MIBRID COLOR POWDER, *15: dimethicone-disodium stearoyl glutamate-composite-treated talc: manufactured by Miyoshi Kasei, Inc., SA/NAI-TA-46R MIBRID COLOR POWDER, *16: dimethicone-treated silica: manufactured by Miyoshi Kasei, Inc., A-SB-300 (7%), *17: calcium carbonate: manufactured by Ohmi Chemical Industry Co., Ltd., light calcium carbonate (volume average particle diameter 7.5 $\mu$m, spindle shape, aspect ratio 1.9) *18: calcium carbonate: manufactured by Sakai Chemical Industry Co., Ltd., SCS-M5, (volume average particle diameter 5 $\mu$m, spherical shape, aspect ratio 1) *19: magnesium carbonate: manufactured by Kyowa Chemical Industry Co., Ltd., heavy magnesium carbonate (volume average particle diameter 16 $\mu$m, plate shape, aspect ratio 26)

Formulation Examples 1 to 4

Water-in-oil emulsion cosmetics having the compositions shown in Table 3 were produced in the same manner as Examples 1 to 13.

The obtained water-in-oil emulsion cosmetics all have no sliding feeling during application, a feeling of close adhesion to the skin after application, excellent pore covering ability, and no stickiness of the skin after application.

[Table 3]

| Component (% by mass) | | | | Formulation Example 1 | Formulation Example 2 | Formulation Example 3 | Formulation Example 4 |
|---|---|---|---|---|---|---|---|
| Oil phase | (A) | Sorbitan monoisostearate | *1 | 0.5 | 0.5 | 0.5 | 0.5 |
| | (A) | Polyoxyethylene-methylpolysiloxane copolymer | *2 | 4 | 4 | 4 | 4 |
| | (B) | Glyceryl tribehenate | *3 | 0.1 | 0.1 | 0.1 | 0.1 |
| | (C) | Liquid isoparaffin | | 0.05 | 0.2 | 0.7 | 1.5 |
| | (C) | Methylphenylpolysiloxane | *6 | 0.36 | 1.5 | 6 | 11 |
| | (C) | Caprylyl methicone | | 0.45 | 1.8 | 7.2 | 13.5 |
| | (C) | Propylene glycol dicaprate | | 0.14 | 0.5 | 2.1 | 4 |
| | | Dimethylpolysiloxane (2cs) | *7 | 5.5 | 5.5 | 5.5 | 5.5 |
| | | Light liquid isoparaffin | *8 | 1.5 | 1.5 | 1.5 | 1.5 |
| | | (Acrylates/polytrimethylsiloxymethacrylate) copolymer-dimethicone mixture (40% by mass dimethylpolysiloxane (2 cs) solution) | *9 | 7 | 7 | 7 | 7 |
| Water phase | | Glycerin | | 1 | 1 | 1 | 1 |
| | | Dipropylene glycol | | 4 | 4 | 4 | 4 |
| | | Sodium chloride | | 0.5 | 0.5 | 0.5 | 0.5 |
| | | Phenoxyethanol | | 0.5 | 0.5 | 0.5 | 0.5 |
| | (E) | Purified water | | 57.9 | 54.9 | 42.9 | 28.9 |

(continued)

| | | Component (% by mass) | | Formulation Example 1 | Formulation Example 2 | Formulation Example 3 | Formulation Example 4 |
|---|---|---|---|---|---|---|---|
| Powder phase | | Dimethicone-disodium stearoyl glutamate-composite-treated titanium oxide | *10 | 10 | 10 | 10 | 10 |
| | | Dimethicone-disodium stearoyl glutamate-composite-treated red oxide | *12 | 0.3 | 0.3 | 0.3 | 0.3 |
| | | Dimethicone-disodium stearoyl glutamate-composite-treated yellow iron oxide | *13 | 1.3 | 1.3 | 1.3 | 1.3 |
| | | Dimethicone-disodium stearoyl glutamate-composite-treated black iron oxide | *14 | 0.15 | 0.15 | 0.15 | 0.15 |
| | | Dimethicone-disodium stearoyl glutamate-composite-treated talc | *15 | 2.25 | 2.25 | 2.25 | 2.25 |
| | | Dimethicone-treated silica | *16 | 0.5 | 0.5 | 0.5 | 0.5 |
| | (D) | Calcium carbonate | *17 | 0.5 | 0.5 | 0.5 | 0.5 |
| | (D) | Magnesium carbonate | *19 | 1.5 | 1.5 | 1.5 | 1.5 |
| | | Total | | 100.00 | 100.00 | 100.00 | 100.00 |
| | | (A) Total amount | | 4.50 | 4.50 | 4.50 | 4.50 |
| | | (B) Total amount | | 0.10 | 0.10 | 0.10 | 0.10 |
| | | (C) Total amount | | 1.00 | 4.00 | 16.00 | 30.00 |
| | | (D) Total amount | | 2.00 | 2.00 | 2.00 | 2.00 |
| | | (B)/(D) | | 0.05 | 0.05 | 0.05 | 0.05 |
| | | (C)/(D) | | 0.50 | 2.00 | 8.00 | 15.00 |

**Claims**

1. A water-in-oil emulsion composition comprising the following components (A), (B), (C), (D), and (E):

   (A) a lipophilic nonionic surfactant;
   (B) an oil agent which is solid or semisolid at 25°C;
   (C) a nonvolatile oil which is liquid at 25°C;
   (D) a carbonate; and
   (E) water.

2. The water-in-oil emulsion composition according to claim 1, wherein a content of the component (A) is from 1 to 8% by mass.

3. The water-in-oil emulsion composition according to claim 1 or 2, wherein a content of the component (B) is from 0.01 to 8% by mass.

4. The water-in-oil emulsion composition according to any one of claims 1 to 3, wherein a content of the component (C) is from 0.5 to 35% by mass.

5. The water-in-oil emulsion composition according to any one of claims 1 to 4, wherein a content of the component (D) is from 0.1 to 10% by mass.

6. The water-in-oil emulsion composition according to any one of claims 1 to 5, wherein the component (A) comprises at least one or more selected from the group consisting of sorbitan fatty acid esters and silicone-based surfactants.

7. The water-in-oil emulsion composition according to any one of claims 1 to 6, wherein the component (B) comprises at least one or more selected from the group consisting of vegetable waxes, phytosterol derivatives, and triglycerides.

8. The water-in-oil emulsion composition according to any one of claims 1 to 7, wherein the component (D) comprises at least one or more selected from the group consisting of calcium carbonate and magnesium carbonate.

9. The water-in-oil emulsion composition according to any one of claims 1 to 8, wherein a mass ratio of the component (B) to the component (D), (B)/(D), is from 0.007 to 50.

10. The water-in-oil emulsion composition according to any one of claims 1 to 9, wherein a mass ratio of the component (C) to the component (D), (C)/(D), is from 0.1 to 100.

11. The water-in-oil emulsion composition according to any one of claims 1 to 10, wherein a content of a coloring pigment is from 1 to 30% by mass.

12. The water-in-oil emulsion composition according to any one of claims 1 to 11, being a water-in-oil emulsion cosmetic.

13. Use of the water-in-oil emulsion composition according to any one of claims 1 to 12 as a makeup cosmetic.

14. Use for makeup of the water-in-oil emulsion composition according to any one of claims 1 to 12, which is applied to a skin, preferably a face.

## INTERNATIONAL SEARCH REPORT

International application No.

**PCT/JP2021/037567**

### A. CLASSIFICATION OF SUBJECT MATTER

*A61K 8/37*(2006.01)i; *A61K 8/06*(2006.01)i; *A61K 8/19*(2006.01)i; *A61K 8/63*(2006.01)i
FI:   A61K8/37; A61K8/06; A61K8/19; A61K8/63

According to International Patent Classification (IPC) or to both national classification and IPC

### B. FIELDS SEARCHED

Minimum documentation searched (classification system followed by classification symbols)

A61K8/37; A61K8/06; A61K8/19; A61K8/63

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2011-1332 A (SHIN-ETSU CHEMICAL CO LTD) 06 January 2011 (2011-01-06) claim 1, paragraphs [0017]-[0019], [0069], [0121], [0179]-[0180] | 1-14 |
| Y | JP 2017-66092 A (KAO CORP) 06 April 2017 (2017-04-06) claims, paragraphs [0007], [0029], table 1 | 1-14 |
| Y | JP 2013-103885 A (KOSE CORP) 30 May 2013 (2013-05-30) paragraphs [0026]-[0028] | 1-14 |
| Y | JP 2010-180205 A (KOSE CORP) 19 August 2010 (2010-08-19) paragraphs [0066]-[0067] | 1-14 |
| A | JP 2016-185919 A (KOSE CORP) 27 October 2016 (2016-10-27) entire text | 1-14 |
| A | JP 2017-178887 A (KOSE CORP) 05 October 2017 (2017-10-05) entire text | 1-14 |

☐ Further documents are listed in the continuation of Box C.      ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **06 December 2021** | **21 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

PCT/JP2021/037567

| Patent document cited in search report | | | Publication date (day/month/year) | Patent family member(s) | Publication date (day/month/year) |
|---|---|---|---|---|---|
| JP | 2011-1332 | A | 06 January 2011 | EP 2266532 A2 claim 1, paragraphs [0016]-[0018], [0069], [0130], [0198]-[0200] | |
| JP | 2017-66092 | A | 06 April 2017 | (Family: none) | |
| JP | 2013-103885 | A | 30 May 2013 | (Family: none) | |
| JP | 2010-180205 | A | 19 August 2010 | (Family: none) | |
| JP | 2016-185919 | A | 27 October 2016 | (Family: none) | |
| JP | 2017-178887 | A | 05 October 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014172872 A
- JP 2011236182 A
- JP 2020506953 A
- JP H111530 A
- JP 2000063225 A
- JP 2025411 A
- JP H4312511 A